# EUROPEAN PATENT APPLICATION

(11) **EP 4 721 653 A1**
(43) Date of publication of application: **08.04.2026**
(21) Application number: 25205160.2
(22) Date of filing: 28.09.2025
(51) Int. Cl.: A61B 5/021, A61B 5/022, A61B 5/0235

(54) **REPLACEABLE EXHAUST PIPE FITTINGS, BLOOD PRESSURE MONITOR FOR MOUNTING THE SAME AND EXHAUST METHOD THEREFORE**

(30) Priority: 01.10.2024 TW 113137666
(71) Applicant: AViTA Corporation, New Taipei City 24158 (TW)
(72) Inventor: CHUANG, Pi-Hao, 24158 New Taipei City (TW); HUANG, I-Chih, 24158 New Taipei City (TW); WU, Yu-Hsin, 24158 New Taipei City (TW)
(74) Representative: Schwerbrock, Florian

(57) **Abstract**

The present invention provides a replaceable exhaust pipe fitting for connecting to an airbag unit, comprising: a body having a air passage; a first conduit extending from said body; and a second conduit extending from said body, wherein said first conduit and said second conduit are interconnected through said air passage, and said second conduit forms at least one vent hole, such that during pressurization of said airbag unit through said air passage, said vent hole functions as a slow-release pressure vent hole. The blood pressure monitor and exhaust method of the present invention utilize the replaceable exhaust pipe fitting to accommodate different sizes of the airbag unit, thereby enabling more accurate measurement results from the blood pressure monitor.

## Description

### BACKGROUND OF THE INVENTION

### 1. FIELD OF THE INVENTION

The present invention relates to a replaceable exhaust pipe fitting for a blood pressure monitor. More specifically, the invention relates to a blood pressure monitor employing such a replaceable exhaust pipe fitting and an exhaust method therefore.

### 2. DESCRIPTION OF THE RELATED ART

Among common lifestyle diseases, hypertension is a chronic condition with high prevalence in modern society. However, control rates remain suboptimal in both Western countries and Taiwan, and hypertension continues to rank among the top ten causes of death. To prevent and treat cardiovascular diseases, blood pressure measurement has become increasingly vital in both clinical and home healthcare settings. Consequently, there is a significant demand for blood pressure monitoring devices that can quickly and accurately measure blood pressure.

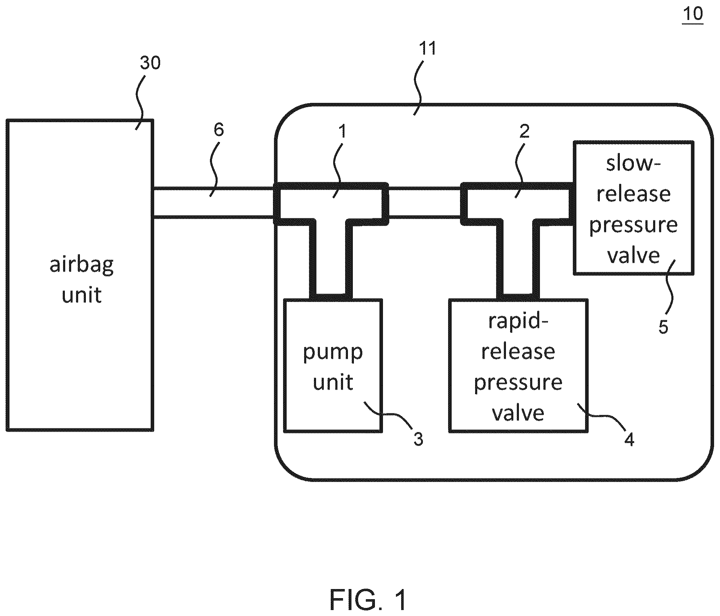

Figure 1 shows a block diagram of the conventional blood pressure monitor equipped with a slow pressure relief valve, illustrating its internal interconnections. The conventional blood pressure monitor 10 comprises a main body 11, an airbag unit 30, and an air pipe 6 connecting the airbag unit 30 to the main body 11. The main body 11 forms a housing space with a connection hole, to which the air pipe 6 is connected. The housing space accommodates a pump unit 3, a rapid-release pressure valve 4, a slow-release pressure valve 5, and a control circuit (not shown) with a pressure sensor. The pump unit 3, rapid-release pressure valve 4, and slow-release pressure valve 5 are interconnected to the connection hole via connecting fittings 1 and 2, and further connected to the cuff unit 30 through the air pipe 6. During blood pressure measurement, the cuff unit 30 is wrapped around a user's arm. The pump unit 3 of the conventional blood pressure monitor 10 rapidly pressurizes the cuff unit 30 to a predetermined value. Subsequently, the slow-release pressure valve 5 of the conventional blood pressure monitor 10 begins gradual deflation. At this point, the conventional blood pressure monitor 10 commences pulse signal measurement. Upon completion of the blood pressure measurement, the rapid-release pressure valve 4 activates to fully release the remaining air.

However, since the circumference of a user's arm may vary from person to person-for example, between the elderly and the young, adults and children, men and women, or arms used for playing badminton and tennis- When different cuff sizes are used for arms of varying thickness, the amount of air injected during the inflation phase of the conventional blood pressure monitor differs. Since the slow-release pressure valve 5 configured for the conventional blood pressure monitor is installed inside the main body 11, replacing the slow-release pressure valve 5 is not easily achievable. Therefore, during the pulse wave signal measurement following inflation, if the cuff relies solely on slow-release pressure valves with identical specifications for deflation, it may result in inconsistent deflation rates or even excessively slow deflation. This could lead to variations in measurement duration and potentially introduce measuring errors in blood pressure measurements across different users.

Utility Model Patent No. M469879 discloses a blood pressure measurement device applicable to different sizes of cuffs. The internal connecting structure of the main body of this blood pressure measurement device is configured with two slow-release pressure valves. By utilizing a connecting structure where the two slow-release pressure valves are either interconnected or not interconnected with each other, and by adapting cuff connectors with different connecting structures to connect the two slow-release pressure valves inside the main body, the blood pressure measurement device can be adapted for different sizes of cuffs. However, this prior art configuration of two slow-release pressure valves within the interconnection structure not only increases the device's overall volume but also prevents replacement of the valves. To accommodate different cuff sizes, the device requires additional internal components and cuff connectors with different connecting structures, further increasing production costs.

Therefore, the present invention provides a suitable for use with replaceable exhaust pipe fittings and an exhaust method therefore. This eliminates the need for a slow-release pressure valve within the interconnecting structure of the blood pressure monitor. By allowing the exhaust pipe fitting to be replaced for different cuff sizes, the production cost of the blood pressure monitor can be reduced. Additionally, the size of the monitor can be minimized for easier portability, demonstrating practical industrial applicability.

### SUMMARY OF THE INVENTION

The purpose of the present invention is to provide a replaceable exhaust pipe fitting that accommodates different sizes of airbag units, thereby eliminating the need for a slow-release pressure valve within the internal connecting structure of the blood pressure monitor unit itself.

Another purpose of the present invention is to provide a blood pressure monitor suitable for use with replaceable exhaust pipe fittings and an exhaust method therefore, enabling the use of different cuff sizes to accommodate variations in user arm circumference while maintaining the accuracy of blood pressure measurements.

To achieve the purposes of the present invention, the present invention provides a replaceable exhaust pipe fitting for connecting an airbag unit, comprising: a body having a air passage; a first conduit extending from said body; and a second conduit extending from said body, wherein said first conduit and said second conduit are interconnected through said air passage, and said second conduit forms at least one vent hole, such that during pressurization of said airbag unit through said air passage, said vent hole functions as a slow-release pressure vent hole.

To achieve the purposes of the present invention, the present invention further provides a blood pressure monitor comprising: a main body having a connection hole and a housing space, wherein the housing space accommodates a pump unit and a rapid-release pressure valve, and the connection hole connects the pump unit and the rapid-release pressure valve; a replaceable exhaust pipe fitting detachably mounted on the connection hole of the main body, wherein the replaceable exhaust pipe fitting has a air passage connecting with the connection hole; and an airbag unit connected to the air passage of the replaceable exhaust pipe fitting; wherein the air passage of the replaceable exhaust pipe fitting forms at least one vent hole, such that during pressurization of the airbag unit by the pump unit, the vent hole functions as a slow-release pressure vent hole.

To achieve the purposes of the present invention, the present invention further provides an exhaust method for a blood pressure monitor, comprising: providing an airbag unit having a predetermined size; in response to said predetermined size of the airbag unit, configuring a replaceable exhaust pipe fitting to connect said airbag unit, wherein the aperture diameter of a vent hole of said replaceable exhaust pipe fitting corresponds to said predetermined size of the airbag unit; mounting the replaceable exhaust pipe fitting onto a connection hole of the blood pressure monitor, such that during pressurization of said airbag unit through said air passage, said vent hole functions as a slow-release pressure vent hole.

The blood pressure monitor and exhaust method implemented according to the present invention utilize replaceable exhaust pipe fittings corresponding to different sizes of the airbag unit. This eliminates the need for a slow-release pressure valve within the interconnecting structure of the blood pressure monitor. Consequently, the production cost of the blood pressure monitor is reduced, and its size is minimized for enhanced portability.

### BRIEF DESCRIPTION OF THE DRAWINGS

FIG. 1 is a block diagram of the conventional blood pressure monitor equipped with a slow-release pressure valve.
FIG. 2 is a block diagram of the blood pressure monitor of the present invention configured with a replaceable exhaust pipe fitting.
FIG. 3A is a side view of the replaceable exhaust pipe fitting of the first embodiment of the present invention.
FIG. 3B is a front view of the replaceable exhaust pipe fitting of the first embodiment of the present invention.
FIG. 4 is a three-dimensional view of the replaceable exhaust pipe fitting of the second embodiment of the present invention.

### DETAILED DESCRIPTION OF THE EMBODIMENTS

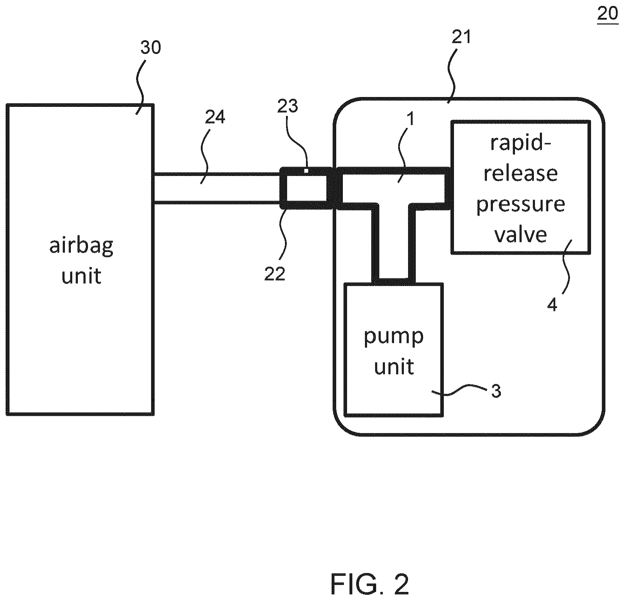

First, refer to FIG. 2, which shows a block diagram of the blood pressure monitor of the present invention configured with a replaceable exhaust pipe fitting. In one embodiment of the present invention, a blood pressure monitor 20 comprises: a main body 21, an airbag unit 30, and a tube 24 connecting the airbag unit 30 to the main body 21, wherein the airbag unit 30 has a predetermined size. The main body 21 forms a housing space with a connecting hole. The air tube 24 is installable to connect to the connecting hole. The housing space accommodates a pump unit 3, a rapid-release pressure valve 4, and a control circuit (not shown) with a pressure sensor, without a slow-release pressure valve. The pump unit 3 and the rapid-release pressure valve 4 are interconnected to the connecting hole via an internal connecting fitting 1, thereby communicating with the airbag unit 30 through the air tube 24.

Referring again to FIG. 2, the blood pressure monitor 20 of the present invention is configured with a replaceable exhaust pipe fitting 22, which includes a air passage. One end of the air passage connects to the air tube 24. When the replaceable exhaust pipe fitting 22 is installed into the connecting hole of the main body 21, the other end of the air passage connects to the internal connecting fitting 1. This allows the air tube 24 to connect to the pump unit 3 and the rapid-release pressure valve 4 via the installation of the replaceable exhaust pipe fitting 22. The replaceable exhaust pipe fitting 22 of the present invention is provided with at least one vent hole 23, allowing the air passage to communicate externally through the vent hole 23. The diameter of the vent hole 23 corresponds to the predetermined size of the airbag unit 30. In different embodiments of the present invention, the predetermined size of the airbag unit 30 is XL or L, wherein both XL and L predetermined sizes are 31.8 cm × 15.7 cm, then the aperture diameter of the vent hole 23 of the replaceable exhaust pipe fitting 22 paired with the airbag unit 30 is 0.21 mm; When the preset size of the airbag unit 30 is M or U, where both M and U preset sizes are 23cm × 12.5cm, the aperture diameter of the vent hole 23 of the replaceable exhaust pipe fitting 22 paired with the airbag unit 30 is 0.15mm; When the preset size of the airbag unit 30 is XS, where XS has a preset size of 14.8 cm × 12 cm, the aperture diameter of the vent hole 23 of the replaceable exhaust pipe fitting 22 paired with the airbag unit 30 is 0.13 mm

Before blood pressure measurement, the predetermined size of the airbag unit 30 corresponding to the user's arm circumference is selected and paired with a replaceable exhaust pipe fitting 22 featuring an aperture diameter of the vent hole 23 corresponding to the predetermined size, thereby connecting the airbag unit 30 to the blood pressure monitor 20 of the present invention. During blood pressure measurement, the airbag unit 30 is wrapped around the user's arm. The pump unit 3 of the blood pressure monitor 20 rapidly pressurizes until the pressure within the airbag unit 30 rises to a predetermined pressure value. During pressurization, the vent hole 23 of the replaceable exhaust pipe fitting 22 functions as a slow-release pressure vent hole. At this point, the blood pressure monitor 20 commences the pulse signal measurement. Upon completion of blood pressure measurement, the rapid-release pressure valve 4 of the blood pressure monitor 20 activates to fully exhaust the remaining gas within the airbag unit 30. Therefore, although the main body 21 of the blood pressure monitor 20 according to the present invention does not include a slow-release pressure valve, the vent hole 23 of the replaceable exhaust pipe fitting 22 functions as a slow-release pressure vent hole during blood pressure measurement, enabling the replaceable exhaust pipe fitting 22 to replace the slow-release pressure valve.

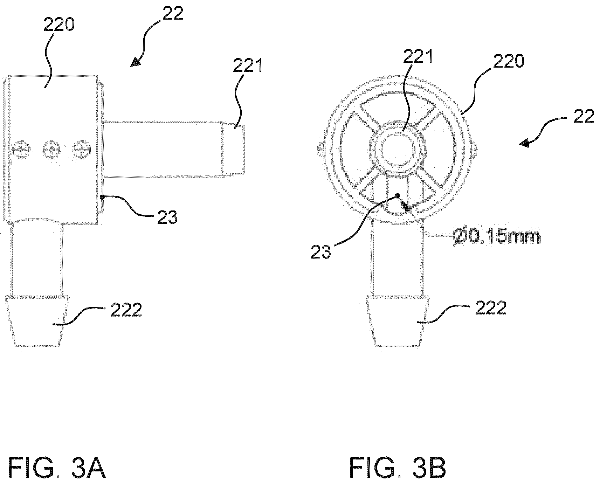

Refer simultaneously to FIGs. 3A and 3B, which respectively show the side view and front view of the replaceable exhaust pipe fitting according to the first embodiment of the present invention. In the first embodiment of the present invention, a replaceable exhaust pipe fitting 22 is provided for connecting to an airbag unit 30, wherein said airbag unit 30 has a predetermined size. The replaceable exhaust pipe fitting 22 comprises a body 220 with an air passage. The body 220 extends a first conduit 221 and a second conduit 222, wherein the first conduit 221 and the second conduit 222 are interconnected via the air passage. The first conduit 221 is inserted into the connecting hole of the blood pressure monitor 20, enabling the air passage to connect the internal connecting structure of the main body 21. The second conduit 222 connects to an air tube 24 of the airbag unit 30, enabling the air passage to connect the airbag unit 30. The replaceable exhaust pipe fitting 22 of the present invention includes at least one vent hole 23. This vent hole 23 has an aperture diameter corresponding to the predetermined size of the airbag unit 30. Taking the predetermined size M or U as an example, the aperture diameter of the vent hole 23 is 0.15 mm.

In different embodiments of the present invention, the vent hole 23 may be provided on the body 220, the first conduit 221, or the second conduit 222 of the replaceable exhaust pipe fitting 22. As shown in FIGs. 3A and 3B, the vent hole 23 is disposed on the body 220. Preferably, the vent hole 23 should be disposed to avoid obstruction by other structural walls or objects after installation, ensuring unimpeded exhaust flow. Furthermore, the first conduit 221 and second conduit 222 of the replaceable exhaust pipe fitting 22 extend from the body 220 in different directions, forming either an L-shaped or straight-through air passage.

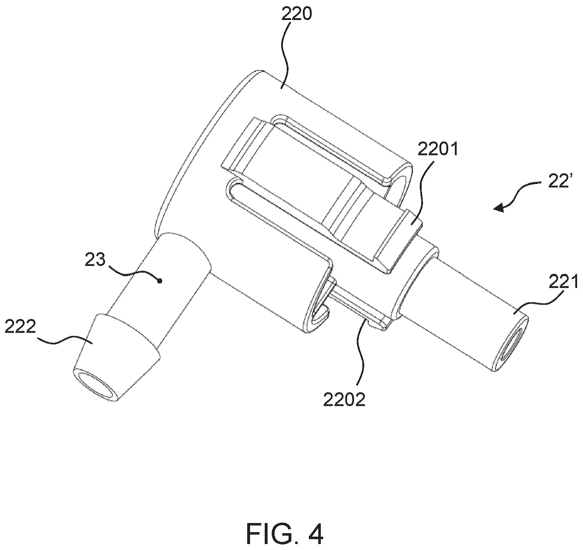

Referring to FIG. 4, it shows a three-dimensional view of the replaceable exhaust pipe fitting 22' according to the second embodiment of the present invention. In the second embodiment of the present invention, a replaceable exhaust pipe fitting 22' is used to connect to an airbag unit 30, wherein the airbag unit 30 has a predetermined size. The replaceable exhaust pipe fitting 22' of the present invention comprises a body 220 as in the first embodiment, a first conduit 221, and a second conduit 222. The first conduit 221 and the second conduit 222 are interconnected through an air passage in the body 220. The vent hole 23 is provided on the side wall of the second conduit 222. Furthermore, the body 220 of the replaceable exhaust pipe fitting 22' of the present invention further forms a pair of retaining arms 2201, 2202, which are preferably disposed on opposite sides of the first conduit 221. The pair of retaining arms 2201, 2202 can be retained on the edge of the connecting hole of the main body 21 of the blood pressure monitor 20 of the present invention to maintain the first conduit 221 within the connecting hole.

A blood pressure monitor 20 implemented according to the first or second embodiment of the present invention, and an exhaust method for the blood pressure monitor comprises the following steps: the blood pressure monitor 20 provides an airbag unit having a predetermined size; In correspondence to the predetermined size of said airbag unit, the blood pressure monitor 20 is equipped with a replaceable exhaust pipe fitting connected to the airbag unit, wherein the replaceable exhaust pipe fitting has at least one vent hole, and the aperture diameter of the vent hole corresponds to the predetermined size of the airbag unit; the replaceable exhaust pipe fitting is installed in a connecting hole on the main body of the blood pressure monitor 20; and during the pressurization of the airbag unit by the blood pressure monitor 20, the vent hole functions as a slow-release pressure vent hole.

## Claims

1. A blood pressure monitor(20), comprising:
a main body(21), having a connecting hole and a housing space, wherein the housing space accommodates a pump unit(3) and a rapid-release pressure valve(4), without a slow-release pressure valve(5), and the pump unit(3) and the rapid-release pressure valve(4) are interconnected to the connecting hole;
a replaceable exhaust pipe fitting(22), detachably mounted on the connecting hole of the main body(21), wherein the replaceable exhaust pipe fitting(22) has an air passage connecting with the connecting hole; and
an airbag unit(30), connected to the air passage of the replaceable exhaust pipe fitting(22);
wherein the air passage of the replaceable exhaust pipe fitting(22) forms at least one vent hole(23), such that during pressurization of the airbag unit(30) by the pump unit(3), the vent hole(23) functions as a slow-release pressure vent hole.

2. The blood pressure monitor(20) of claim 1, wherein the airbag unit(30) has a predetermined size, and when paired with the replaceable exhaust pipe fitting(22), the aperture of the vent hole(23) corresponds to the predetermined size of the airbag unit(30).

3. The blood pressure monitor(20) of claim 2, wherein the predetermined size is 31.8 cm × 15.7 cm, and the aperture diameter of the vent hole(23) is 0.21 mm.

4. The blood pressure monitor(20) of claim 2, wherein the predetermined size is 23 cm × 12.5 cm, and the aperture diameter of the vent hole(23) is 0.15 mm.

5. The blood pressure monitor(20) of claim 2, wherein the predetermined size is 14.8 cm × 12 cm, and the aperture diameter of the vent hole(23) is 0.13 mm.

6. A replaceable exhaust pipe fitting(22) for connecting an airbag unit(30), comprising:
a body(220), having an air passage;
a first conduit(221), extending from said body(220); and
a second conduit(222), extending from said body(220),
wherein said first conduit(221) and said second conduit(222) are interconnected through said air passage, and the replaceable exhaust pipe fitting(22) forms at least one vent hole(23), such that during pressurization of said airbag unit(30) through said air passage, said vent hole(23) functions as a slow-release pressure vent hole.

7. The replaceable exhaust pipe fitting(22) of claim 6, wherein the airbag unit(30) has a predetermined size, and when paired with the replaceable exhaust pipe fitting(22), the aperture of the vent hole(23) corresponds to the predetermined size of the airbag unit(30).

8. The replaceable exhaust pipe fitting(22) of claim 6, wherein the vent hole(23) disposes in the body(220), the first conduit(221), or the second conduit(222), thereby enabling the air passage to communicate with the exterior through the vent hole(23).

9. The replaceable exhaust pipe fitting(22) of claim 8, wherein the body(220) forms a pair of retaining arms(2201,2202) that engage a connecting hole of a blood pressure monitor to maintain the first conduit(221) within the connecting hole.

10. An exhaust method for the blood pressure monitor(20) of claim 1, comprising:
providing an airbag unit(30) having a predetermined size;
in response to said predetermined size of the airbag unit(30), configuring a replaceable exhaust pipe fitting(22) to connect said airbag unit(30), wherein the aperture diameter of a vent hole(23) of said replaceable exhaust pipe fitting(22) corresponds to said predetermined size of the airbag unit(30);
mounting the replaceable exhaust pipe fitting(22) onto a connecting hole of the blood pressure monitor(20), such that during pressurization of said airbag unit(30) through said air passage, said vent hole(23) functions as a slow-release pressure vent hole.
